# DEMANDE DE BREVET EUROPEEN

(11) **EP 1 382 354 A1**
(43) Date de publication de la demande: **21.01.2004**
(21) Numéro de dépôt: 03291786.6
(22) Date de dépôt: 18.07.2003
(51) Int. Cl.: A61L 2/23, A01N 1/02, A23C 3/08

(54) **Composition nettoyante, detergente, desinfectante et decontaminante, a large spectre d'utilisation**

(30) Priorité: 19.07.2002 FR 0209237
(71) Demandeur: Guichard, Philippe, 54290 Lorey (FR); Braun, Jean-Paul, 67000 Strasbourg (FR); Arth, Guy, 57680 Le Mud (FR); Guenois, Gilles, 88130 Charmes (FR); Weiss, Olivier, 88500 Mericourt (FR)
(72) Inventeur: Guichard, Philippe, 54290 Lorey (FR); Wackermann, Guy, 67000 Strasbourg (FR)
(74) Mandataire: Gallochat, Alain

(57) **Abrégé**

La présente invention concerne une nouvelle composition nettoyante, détergente, désinfectante et décontaminante, à large spectre d'utilisation.

Selon un mode préférentiel de l'invention, la composition selon l'invention comprend notamment du tripolyphosphate de sodium et un agent oxydant, le rapport pondéral du tripolyphosphate de sodium par rapport à l'agent oxydant se situant entre 2 :1 et 3 :1.

Application aux domaines agro-alimentaire, médical, hospitalier, notamment en matière de soin et d'hygiène.

## Description

La présente invention concerne une nouvelle composition nettoyante, détergente, désinfectante et décontaminante, à large spectre d'utilisation.

Il est connu des compositions détergentes ayant essentiellement la vocation de blanchir et de nettoyer des textiles ; de telles compositions ne sont toutefois pas valables dans les domaines touchant à l'alimentaire ou à l'humain.

La composition faisant l'objet de la présente invention répond aux impératifs liés à ces domaines.

De façon plus précise, la composition nettoyante, détergente, désinfectante et décontaminante selon l'invention, du type à base de tripolyphosphate de sodium et d'un agent oxydant, présentera un rapport pondéral du tripolyphosphate de sodium par rapport à l'agent oxydant se situant entre 2 :1 et 3 :1 et préférentiellement de 2,5 :1.

Selon un mode préférentiel de l'invention, l'agent oxydant sera le percarbonate de sodium et le tripolyphosphate de sodium se présentera sous forme préhydratée.

Dans une variante préférée de l'invention, la composition nettoyante, détergente, désinfectante et décontaminante, comportera notamment en poids :
- 15 - 25% de percarbonate de sodium
- 45 - 58% de tripolyphosphate de sodium préhydraté

D'autres constituants pourront être ajoutés à la composition selon l'invention ; cette composition se présentera sous forme de poudre, éventuellement compactée en une pastille. Après dilution dans un liquide tel que l'eau, le pH de la solution, résultant des constituants de la composition et de leurs proportions dans cette dernière, se situera aux environs de 9, ce qui conférera à cette solution un effet bactéricide appréciable.

Outre le percarbonate de sodium, ou autre agent oxydant, et le tripolyphosphate de sodium préhydraté, il sera avantageux d'adjoindre à la composition un activateur de l'agent oxydant, tel que la tétraacétyléthylène diamine (TAED) préférentiellement sous forme enrobée à 90% ; dans ce cas, un tel activateur sera présent dans la composition à hauteur de 11 - 14% en poids.

Selon les qualités requises pour la composition, il pourra être envisagé de lui adjoindre du polyéthylène glycol (PM 4000) à raison de 6,5 - 8,5% en poids et du citrate trisodique sous forme de dihydrate dans les mêmes proportions ; un agent anti-moussant tel qu'un alcool gras (alcool gras éthoxylépropoxylé par exemple) pourra également faire partie de la composition.

Ainsi, à titre préférentiel, la présente invention concerne une composition nettoyante, détergente, désinfectante et décontaminante comportant notamment, en poids :
- 0,2 - 0,3% d'alcool gras éthoxylé propoxylé
- 11 - 14% de TAED enrobée à 90%
- 18 - 22% de percarbonate de sodium
- 46 - 57% de tripolyphosphate de sodium préhydraté.

Comme cela a été mentionné plus haut, on intégrera avantageusement à cette composition 6,5 - 8,5 % en poids de polyéthylène glycol (PM 4000) et/ou autant de citrate trisodique sous forme de dihydrate ; à titre indicatif, une pastille de 8, 16 ou 32 grammes préparée à partir des divers composants précités dans les proportions indiquées correspond au traitement de ½, 1 ou 2 litres, soit 16 grammes par litre.

Cette composition se présentera naturellement sous forme pulvérulente, mais il sera également possible de la compacter afin d'obtenir une pastille comme cela vient d'être décrit ; elle pourra alors être dissoute dans de l'eau et permettre par trempage de nettoyer couteaux et instruments de chirurgie ou par brumisation de nettoyer toutes surfaces ou volumes.

Une autre utilisation pourra être en thanatopraxie, en diluant la composition selon l'invention dans le sang ou autres liquides biologiques.

Grâce aux propriétés de la composition selon l'invention, il sera également possible de l'utiliser dans les eaux blanches de rinçage de circuit de lait.

La composition selon l'invention présente de nombreuses qualités :
- effet anti-odeur immédiat
- effet désinfectant, dégraissant, nettoyant et décontaminant
- effet neutralisant les protéines et la majeure partie des souches virales et microbiennes (en raison principalement de son pH de l'ordre de 9 en solution).

Ainsi, diverses études ont été menées confirmant l'effet bactéricide et virucide de la composition selon l'invention.

### Effet bactéricide

La composition selon l'invention a fait l'objet d'une détermination de l'activité bactéricide dans les conditions d'usage général selon la norme NF EN 1276 d'octobre 1997 ; conformément à cette norme, en 15 minutes à 40°C et en condition de saleté (3g/l d'albumine bovine), la composition selon l'invention, lorsqu'elle est diluée à 3,2 g/l (P/V) dans l'eau dure, présente une activité bactéricide vis-à-vis des souches de référence suivantes: *Pseudomonas aeruginosa* CEP 103467, *Escherichia coli* CIP 54.127, *Staphylococcus aureus* CIP 4.83 et *Enterococcus hirae* CIP 58.55.

Les résultats de cet essai, réalisé par le Laboratoire Santé Environnement Hygiène de Lyon, figurent dans le tableau ci-dessous.

| Souche-test | Réduction ( R ) du nombre de cellules viables |
|---|---|
| *Pseudomonas aeruginosa* | R : > 2,6 10 |
| *Escherichia coli* | R : > 3,1 10 |
| *Staphylococcus aureus* | R : > 1,1 10 |
| *Enterococcus hirae* | R : > 2,8 10 |

L'Institut de Recherche Microbiologique a pour sa part mené deux études ayant abouti aux conclusions suivantes :
- la composition selon l'invention est bactéricide selon la norme européenne NF EN 1040 (avril 1997), en 5 minutes de contact à 20°C sur les souches de référence *Pseudomonas aeruginosa* ATCC 15442 et *Staphylococcus aureus* ATCC 6538 à une concentration en solution mère de 40% (v/v) soit en masse à 8g/l (1/2 pastille par l) : ce produit est donc conforme à la norme précitée à la concentration d'emploi préconisée (soit 16g/l), avec une marge de sécurité de 2;
- la composition selon l'invention présente à 20°C une activité bactéricide spectre 4 *(Pseudomonas aeruginosa* CIP A22, *Escherichia coli* CIP 54 127, *Staphylococcus aureus* CIP 53 154, *Enterococcus hirae* CIP 58 55) en présence d'un mélange d'albumine et d'extrait de levure conforme à la norme AFNOR NF T 72-171 (novembre 1988) à une concentration en solution mère de 25% (v/v) soit en masse à 8 g/l (1/2 pastille /l) : ce produit est donc conforme à la norme précitée en présence de protéines à la concentration préconisée d'une pastille/l (soit 16g/l).

### Effet virucide

L'étude d'une forme concentrée de la composition selon l'invention a été menée au CEA (Laboratoire SPI/BIO Dr. Pascal CLAYETTE ; Pr. Dominique DORMONT), vis-à-vis d'une souche d'ESB au travers de la détection de la PrPres par Western Blot ; deux protocoles expérimentaux (PrPres diluée au 20^{ème} et PrPres non diluée) ont abouti au même résultat, à savoir un effet significatif de la composition selon l'invention, cette dernière étant capable de diminuer d'au moins 4 logarithmes de base 10 la quantité de PrPres détectable.

De plus les constituants de cette composition rendent cette dernière non toxique et sans danger pour les opérateurs, non irritante pour la peau et de manipulation très simple. En outre, les propriétés précitées ne sont pas altérées dans le temps au stockage.

La composition selon l'invention, compte tenu de ses avantages et de ses qualités, trouve ses applications dans un large spectre d'activités :
- agro-alimentaire
- médical
- laboratoire
- vétérinaire
- hospitalier
- soin
- hygiène (notamment les changes, en particulier à usage humain)
- désinfection de surfaces et de volumes.

La présente demande ne se limite pas aux exemples de constituants décrits, mais s'étend à tous produits qui rempliraient la même fonction et aboutiraient au même résultat.

## Revendications

1. Composition nettoyante, détergente, désinfectante et décontaminante, du type comprenant notamment du tripolyphosphate de sodium et un agent oxydant **caractérisée en ce que** le rapport pondéral du tripolyphosphate de sodium par rapport à l'agent oxydant se situe entre 2 :1 et 3 :1.

2. Composition selon la revendication 1 **caractérisée en ce que** le rapport pondéral du tripolyphosphate de sodium par rapport à l'agent oxydant est de l'ordre de 2,5 :1.

3. Composition selon l'une des revendications 1 ou 2 **caractérisée en ce que** l'agent oxydant est le percarbonate de sodium.

4. Composition selon l'une quelconque des revendications 1 à 3, **caractérisée en ce que** le tripolyphosphate est préhydraté.

5. Composition selon les revendications 3 et 4 **caractérisée en ce qu'**elle comprend notamment en poids :
• 15 - 25% de percarbonate de sodium
• 45 - 58% de tripolyphosphate de sodium préhydraté

6. Composition selon la revendication 5 **caractérisée en ce qu'**elle comprend en outre un activateur du percarbonate de sodium tel que la Tétra Acétyl Etylène Diamine (TAED) préférentiellement enrobée à 90%.

7. Composition selon la revendication 6 **caractérisée en ce qu'**elle comprend en outre un agent anti-moussant tel qu'un alcool gras.

8. Composition selon la revendication 7 **caractérisée en ce qu'**elle comprend notamment, en poids :
• 0,2 - 0,3% d' alcool gras éthoxylé propoxylé polyéthylène
• 11 - 14% de TAED enrobée à90 %
• 18 - 22% de percarbonate de sodium
• 46 - 57% de tripolyphosphate de sodium préhydraté

9. Composition selon la revendication 8 **caractérisée en ce qu'**elle comprend en outre en poids 6,5 - 8,5% de polyéthylène glycol PM 4000 et/ou 6,5 - 8,5 % de citrate trisodique sous forme de dihydrate.

10. Composition selon la revendication 9 **caractérisée en ce qu'**elle se présente sous forme de poudre, éventuellement compactée en une pastille.
